(19) Europäisches Patentamt
European Patent Office
Office européen des brevets

(11) Veröffentlichungsnummer : **0 064 667**
**B1**

(12) **EUROPÄISCHE PATENTSCHRIFT**

(45) Veröffentlichungstag der Patentschrift : **28.11.84**

(21) Anmeldenummer : **82103589.6**

(22) Anmeldetag : **28.04.82**

(51) Int. Cl.³ : **C 07 D277/64**, C 07 D215/12, C 07 D235/08, C 07 D263/56, C 07 D263/60, C 07 D263/62, C 07 D277/84, C 07 D417/06, C 07 D421/06, C 09 B 23/00// C07C143/11

# (54) Verfahren zur Herstellung von Sulfoalkylquartärsalzen.

(30) Priorität : **09.05.81 DE 3118374**

(43) Veröffentlichungstag der Anmeldung : **17.11.82 Patentblatt 82/46**

(45) Bekanntmachung des Hinweises auf die Patenterteilung : **28.11.84 Patentblatt 84/48**

(84) Benannte Vertragsstaaten : **CH DE FR GB IT LI**

(56) Entgegenhaltungen :
**DD-A- 139 577**
**DE-A- 2 803 493**
**DE-A- 2 909 200**
**FR-A- 2 393 797**

(73) Patentinhaber : **AGFA-GEVAERT Aktiengesellschaft**
**D-5090 Leverkusen 1 (DE)**

(72) Erfinder : **Kampfer, Helmut, Dr.**
**Roggendorfstrasse 63**
**D-5000 Köln 80 (DE)**
Erfinder : **Himmelmann, Wolfgang, Dr.**
**Am Ziegelfeld 7**
**D-5090 Leverkusen 3 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft ein Verfahren zur Herstellung von Sulfoalkylquartärsalzen tertiärer Amine, insbesondere stickstoffhaltiger heterocyclischer Basen.

Derartige Quartärsalze, die positiv und negativ geladene, durch kovalente Bindungen miteinander verbundene Gruppen enthalten, werden auch als Betaine bezeichnet. Sie spielen in einer Reihe von technischen Prozessen eine wichtige Rolle. Dabei werden diese Betaine entweder als solche direkt verwendet, z. B. in der Galvanotechnik, oder als Zwischenprodukte weiteren Umsetzungen unterworfen. Zur Verwendung der Sulfoalkylbetaine als Zwischenprodukte ist es häufig technisch vorteilhaft, diese nicht erst zu isolieren, sondern die weitere Umsetzung in einem Arbeitsgang an deren Herstellung anzuschließen. Als Zwischenprodukte spielen die Sulfoalkylbetaine beispielsweise eine bedeutende Rolle für die Synthese der als spektrale Sensibilisierungsfarbstoffe für lichtempfindliche Materialien, insbesondere für fotografische Silberhalogenidemulsionen, verwendeten Polymethinfarbstoffe. Insoweit ist ein weiterer Gegenstand der Erfindung die Umsetzung der heterocyclischen Basen über die Sulfoalkylquartärsalze zu Polymethinfarbstoffen.

Verfahren zur Herstellung von Sulfoalkylquartärsalzen tertiärer Amine sind schon lange bekannt. Man setzt die tertiäre Base mit einem Sulfoalkylierungsmittel, für gewöhnlich bei erhöhter Temperatur, um. Als Sulfoalkylierungsmittel sind beschrieben u. a. Halogenalkansulfonsäuren, z. B. 2-Bromethansulfonsäure in US 2 503 776, Na-Iod-ethansulfonat in BE 669 308, Na-Iodbutansulfonat is US 2 912 329 oder 3-Chlor-2-hydroxypropansulfonsäure in DE-AS 1 177 482. Nachteilig an diesen Sulfoalkylierungsmitteln ist der bei Verwendung der freien Sulfonsäuren zum Abfangen des entstehenden Halogenwasserstoffs erforderliche Überschuß an tertiärer Base. Ferner sind Sultone als Sulfoalkylierungsmittel bekannt, so ist die Verwendung von Propan-, Butan-, und Isopentansulton in DE-PS 929 080, von Propensulton in DE-AS 1 447 579 und von 2-Chlorpropansulton in GB 1 090 626 beschrieben. Nachteilig an den Sultonen ist deren z. T. schädliche physiologische Wirkung, so daß ihre Verwendung zum Umweltrisiko und zum Sicherheitsrisiko für die damit umgehenden Personen werden kann.

In neuerer Zeit sind Sulfoalkylierungsmittel beschrieben worden, die die Verwendung cancerogener Sultone vermeiden. So werden in DE-OS 2 825 246 und in Research Disclosure 16374 (November 1977) Hydroxyalkansulfonsäuren und ihre Salze sowie in DD 139 577 wäßrige Lösungen von Hydroxyalkansulfonsäuren und deren O-Alkyl- und O-Acylderivaten als Quaternierungsmittel erwähnt. Nachteilig sind die bei der Reaktion entstehenden erheblichen Wassermengen, die die Quaternierung behindern und z. T. zu niedrigen Ausbeuten führen bzw. durch azeotrope Destillation mit geeigneten Lösungsmitteln entfernt werden müssen.

Ferner wurden O-Sulfoalkylimidoester in Research Disclosure 18040 (April 1979) und damit verwandte O-Sulfoalkylisouroniumbetaine in DE-OS 2 909 200 beschrieben. Auch diese neuen Quarternierungsmittel sind mit einer Reihe von Nachteilen behaftet. So ist ihre Herstellung durch Verwendung von Carbodiimiden, Säurenitrilen bzw. Dialkylharnstoffen relativ teuer. Ihre thermische Stabilität ist begrenzt. Die teilweise hohe Schmelzpunkte zeigenden Verbindungen zwingen zur Verwendung von Lösungsmitteln als Reaktionsmedium.

Aus diesen Gründen lag der Erfindung die Aufgabe zugrunde, ein Verfahren zur Herstellung von Sulfoalkylquartärsalzen zu schaffen, das die genannten Nachteile nicht zeigt.

Es wurde nun ein Verfahren zur Herstellung von Sulfoalkylquartärsalzen tertiärer Amine gefunden, das gekennzeichnet ist durch die Umsetzung eines tertiären Amins mit einem Sulfoalkyl-aryl-ether der allgemeinen Formel I

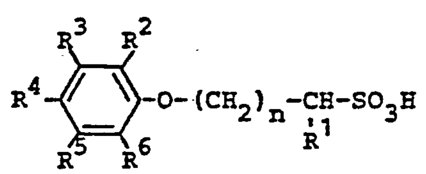

(I)

worin bedeuten

$R^1$ Wasserstoff oder Methyl ;

$R^2$, $R^3$, $R^4$, $R^5$, $R^6$ gleich oder verschieden, und zwar Wasserstoff, Alkyl mit 1 oder 2 C-Atomen, Halogen, z. B. F, Cl, Br oder I, Alkoxy vorzugsweise $OCH_3$, Cyan oder Nitro, (wobei jedoch nicht mehr als zwei Nitrogruppen am Phenylring vorhanden sind) oder $R^2$ und $R^3$ oder $R^3$ und $R^4$ bedeuten jeweils zusammen die zur Vervollständigung eines ankondensierten gesättigten oder ungesättigten 5- oder 6-gliedrigen, gegebenenfalls auch Sauerstoff- oder Schwefelatome enthaltenden Ringes erforderlichen Ringglieder ;

$n = 1$, 2 oder 3.

Die Umsetzung erfolgt bei einer Temperatur zwischen 100 und 250 °C, vorzugsweise bei einer Temperatur zwischen 140 und 180 °C.

Im allgemeinen läuft die Reaktion innerhalb des zuletzt genannten Temperaturbereiches glatt ab. Es

kann jedoch, etwa bedingt durch die Natur eines verwendeten Lösungsmittels, auch außerhalb dieses Temperaturbereiches gearbeitet werden.

An tertiären Aminen sind geeignet prinzipiell alle Derivate des Ammoniak ($NH_3$), in denen jedes der drei Wasserstoffatome substituiert ist, z. B. durch ein Kohlenstoffatom eines Alkyl- oder Arylrestes oder durch ein Kohlenstoffatom oder ein Heteroatom eines heterocyclischen Ringes, wobei insbesondere das Stickstoffatom des tertiären Amins in den heterocyclischen Ring einbezogen sein kann. So sind besonders bevorzugt geeignet heterocyclische Basen der allgemeinen Formel II

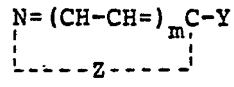

(II)

worin Z die zur Ergänzung einer heterocyclischen Gruppe mit mindestens einem 5- oder 6-gliedrigen heterocyclischen Ring erforderlichen Glieder bedeuten ; der Heteroring kann dabei ankondensierte Benzol-, Naphthalin- oder auch heterocyclische Ringe enthalten, die auch weiter substituiert sein können ; in Frage kommen die aus der Klasse der Cyaninfarbstoffe bekannten Heterocyclen, wie z. B. : Pyrrolin (z. B. 4,4-Dimethyl-pyrrolin), Oxazolin (z. B. 4,4-Dimethyloxazolin), Thiazolin (z. B. 5-Methylthiazolin), Selenazolin, Indolin (z. B. 3,3-Dimethylindolin, 3,3-Dimethyl-5-methoxyindolin, 3,3-Dimethyl-5-diethylamino-indolin), Benzimidazol (z. B. 1-Ethyl-5-trifluormethylbenzimidazol, 1-Methyl-5-chlorbenzimidazol, 1-Ethyl-5,6-dichlor-benzimidazol, 1-Ethyl-5-cyanbenzimidazol, 1-Methyl-5-carbethoxybenzimidazol, 1-Ethyl-5-acetylbenzimidazol, 1-Methyl-benzimidazol-5-sulfonsäurepyrrolidid, 1-Ethyl-benzimidazol-5-sulfonsäuredimethylamid, 1-Ethyl-5-phenylthiobenzimidazol, 1-Methyl-5-methylthiobenzimidazol, 1-Methyl-5-chlor-6-methyl-thiobenzimidazol), Oxazol (z. B. 4-Methyloxazol, 4,5-Diphenyloxazol, 4-Methyl-5-carbethoxyoxazol, Benzoxazol, 5-Chlorbenzoxazol, 5-Phenylbenzoxazol, 6-Methoxybenzoxazol, 5-Methoxybenzoxazol, 5-Methyl-6-methoxybenzoxazol, 5-Brombenzoxazol, 5-Iodbenzoxazol, Naphtho-[2,1-d]-oxazol, Naphtho-[1,2-d]-oxazol, Naphtho-[2,3-d]-oxazol, 4,5,6,7-Tetrahydrobenzoxazol, Benzofuro-[2,3-f]-benzoxazol), Thiazol (z. B. 4-Methyl-thiazol, 4-Phenylthiazol, 4-Methyl-thiazol-5-acrylsäureethylester, Benzthiazol, 5-Methylbenzthiazol, 6-Methylbenzthiazol, 5-Chlorbenzthiazol, 5-Methoxybenzthiazol, 6-Methoxybenzthiazol, 5,6-Dimethylbenzthiazol, 5,6-Dimethoxybenzthiazol, 5-Methyl-6-methoxybenzthiazol, 5-Brombenzthiazol, 5-Phenylbenzthiazol, 6-Methylthiobenzthiazol, 6-Dimethylaminobenzthiazol, 5-Chlor-6-methoxybenzthiazol, 5,6-Methylendioxybenzthiazol, 6-β-Cyanethoxybenzthiazol, 5-Carbomethoxybenzthiazol, 5-Nitro-benzthiazol, 5-Phenylthiobenzthiazol, 5-Thienylbenzthiazol, 6-Hydroxybenzthiazol, 4,5,6,7-Tetrahydrobenzthiazol, 4-Oxo-4,5,6,7-tetrahydrobenzthiazol, Naphtho-[2,1-d]-thiazol, Naphtho-[1,2,d]-thiazol, 4,5-Dihydronaphtho-[1,2-d]-thiazol, 5-Methoxynaphtho-[1,2-d]-thiazol, 5,7,8-Trimethoxy-naphtho-[1,2-d]-thiazol), Selenazol (z. B. Benzselenazol, 5-Methylbenzselenazol, 5,6-Dimethylbenzselenazol, 5-Methoxybenzselenazol, 5-Methyl-6-methoxybenzselenazol, 5,6-Dimethoxybenzselenazol, 5,6-Methylendioxybenzselenazol, 6-Methyl-benzselenazol, Naphtho-[1,2-d]-selenazol, 1,3,4-Oxadiazol (z. B. 5-Methyl-1,3,4-oxadiazol, 5-Phenyl-1,3,4-oxadiazol, 1,3,4-Thiadiazol (z. B. 5-Methyl-1,3,4-thiadiazol, 2,5-Bis-methylthio-1,3,4-thiadiazol, 5-Benzylthio-1,3,4-thiadiazol, 2-Mercapto-5-methylthio-1,3,4-thiadiazol, 5-Carbethoxymethylthio-1,3,4-thiadiazol), Pyridin (z. B. 2-Methylpyridin, 4-Methylpyridin), Pyrimidin (z. B. 2-Methyl-4-methylthiopyrimidin), Chinolin (z. B. 6-Methylchinolin, 6-Methoxychinolin, 8-Chlorchinolin, 6-Fluorchinolin, 5,6-Benzochinolin, 6,7,-Benzochinolin), Imidazol-[4,5-b]-chinoxalin.

$m = 0$ oder 1

Y = bedeutet Wasserstoff, Halogen, eine gesättigte oder ungesättigte aliphatische Gruppe, insbesondere mit bis zu 6 C-Atomen, gegebenenfalls substituiert, z. B. Methyl, Ethyl, Allyl, Cyanalkyl, Halogenalkyl oder Alkoxyalkyl ; Alkoxy, z. B. Carboxyalkoxy, Alkylthio, z. B. Carboxyalkylthio, Sulfoalkylthio, Carbalkoxyalkylthio, oder Mercapto.

Y kann beispielsweise weiterhin eine Methinkette mit 1, 3 oder 5 Methingruppen bedeuten, an deren Ende sich meist über die 2-Stellung angeknüpft eine N-alkylierte heterocyclische Base befindet, wie sie in der Chemie der Cyaninfarbstoffe bekannt sind. Hierzu sei verwiesen auf F.M. Hamer, « The Cyanine Dyes and Ralated Compounds », (1964), Interscience Publishers John Willey and Sons. Verbindungen der Formel II, in der Y in der erwähnten Weise definiert ist, werden als « entquaternierte Cyaninfarbstoffe » bezeichnet. Falls solche entquaternierte Cyaninfarbstoffe nach dem Verfahren der Erfindung umgesetzt werden, sind die unmittelbaren Verfahrensprodukte ohne weitere Umsetzung als Sensibilisierungsfarbstoffe geeignet.

Die Umsetzungen werden im allgemeinen ohne Lösungsmittel durchgeführt, können jedoch auch in Gegenwart eines geeigneten Lösungsmittels vorgenommen werden. Als Lösungsmittel eignen sich alle in bezug auf die erfindungsgemäße Umsetzung inerten Lösungsmittel mit hohem Lösungsvermögen für die Reaktionspartner , wie z. B. Phenol oder m-Kresol sowie m-Xylol, Chlorbenzol, Anisol, aber auch Eisessig und Acetanhydrid. Gelegentlich erweist es sich als vorteilhaft zunächst das Sulfoalkylierungsmittel auf erhöhte Temperatur zu bringen, z. B. auf eine Temperatur zwischen 100 und 250 °C, vorzugsweise zwischen 140 und 180 °C und erst dann, nach Zugabe des zu sulfoalkylierenden tertiären Amins, die Reaktion in der beschriebenen Weise durchzuführen.

Die erfindungsgemäßen Umsetzungen erfolgen unter Abspaltung von einem Phenol der Formel

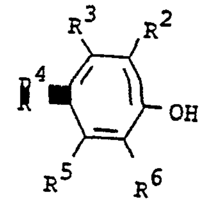

in der die Reste $R^2$, $R^3$, $R^4$, $R^5$, und $R^6$ die bereits angegebene Bedeutung haben. In den meisten Fällen werden die erfindungsgemäßen Ether mit der Basen im Molverhältnis 1 : 1 umgesetzt, wobei pro Mol Base 1 Mol Phenol gebildet wird. Die Umsetzung kann aber auch in anderen Molverhältnissen — z. B. 2 : 1 — durchgeführt werden. Das gebildete Phenol wirkt wegen seines guten Lösungsvermögens für die entstehenden Quartärsalze einer Erstarrung des Reaktionsgemisches entgegen, wodurch die technische Anwendung des Verfahrens sehr erleichtert wird. Ein Überschuß an gebildetem Phenol kann auch aus dem Reaktionsgefäß entfernt werden, beispielsweise durch

1) Arbeiten unter Vakuum,
2) Einleiten eines trockenen Inertgases, z. B. Stickstoff,
3) Verdampfen und Ausfrieren in einer Gefrierapparatur.

Die nach dem Verfahren der Erfindung hergestellten Sulfobetaine tertiärer Amine sind insbesondere solche der folgenden Formel III

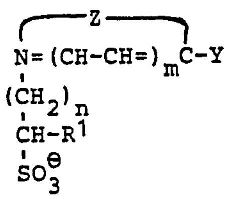

(III)

worin n, m, Y, Z und $R^1$ die bereits angegebene Bedeutung haben.

Diese Verbindungen finden unterschiedliche Verwendung. Sie sind beispielsweise als Leitsalze in der Galvanotechnik geeignet. Desweiteren handelt es sich bei diesen Verbindungen bei geeigneter Bedeutung von Y, d. h. wenn Y, wie bereits erwähnt, eine Methinkette mit 1, 3 oder 5 Methingruppen, an deren Ende sich eine N-alkylierte heterocyclische Base befindet, bedeutet, um die Endprodukte einer Cyaninfarbstoffsynthese. Derartige Verbindungen finden unmittelbar Verwendung für die spektrale Sensibilisierung von lichtempfindlichen Silberhalogenidemulsionen. Aber auch als Zwischenprodukte bei der Synthese von Polymethinfarbstoffen sind die nach dem Verfahren gemäß der Erfindung hergestellten Verbindungen wichtig. So werden beispielsweise die nach dem erfindungsgemäßen Verfahren hergestellten Sulfoalkylquartärsalze heterocyclischer Basen mit Vorteil nicht isoliert, sondern nach Beendigung der Quaternierungsreaktion ohne weitere Reinigungsoperation in bekannter Weise weiter zu Polymethinfarbstoffen umgesetzt.

Die erfindungsgemäß verwendeten Ether I sind im Prinzip bereits bekannt. Sie lassen sich herstellen aus Phenolen und entsprechenden Sultonen durch Erhitzen auf 130 bis 140 °C (vgl. K. Furukawa et al., Kôgyô Kagaku Zasshi *59*, 221-224 (1956), oder aus den Phenolen und Sultonen in Gegenwart von Alkalihydroxid in alkoholischer Lösung (vgl. J.H. Helberger et al., Liebigs Ann. Chem. *565*, 28 (1949), *586*, 147, 150, 157, 163 (1954)). Eine weitere Synthesemethode ist die Umsetzung von Halogenalkansulfonsäuren mit Phenolen in Gegenwart von Alkali.

Herstellungsbeispiel 1

(3-Sulfopropyl)-phenyl-ether (Ether (1)).

60 g Natriumphenolat werden in 250 ml Ethanol innerhalb 1 Stunde mit 60 g Propansulton in 350 ml Ethanol versetzt. Das Reaktionsgemisch erwärmt sich hierbei auf 30 °C und wird dann noch 1 Stunde bei 60 bis 65 °C gerührt. Nach dem Abkühlen wird abgesaugt und mit Ethanol nachgewaschen. Ausbeute 90 g Na-Salz des Ethers (1) = 85 % der Theorie.

Die freie Sulfonsäure wird aus der wäßrigen Lösung des Na-Salzes über Kationenaustauscher gewonnen. Sie wird bei 100 °C im Vakuum getrocknet und gibt ein kristallisierendes Öl. Fp. 70 bis 72 °C.

Herstellungsbeispiel 2

(3-Sulfopropyl)-(3-methylphenyl)-ether (Ether (2)).

108 g m-Kresol werden in 250 ml Methanol und 56 g Kaliumhydroxid bis zur Lösung gerührt. Nach Zugabe von 122 g Propansulton in 500 ml Methanol wird noch 1 Stunde gerührt, dann abgekühlt und abgesaugt. Man wäscht mit Methanol nach. Ausbeute : 83,5 % = 192 g K-Salz des Ethers (2). Durch lösen

des K-Salzes in Wasser und Beschicken einer Kationenaustauschersäule wird eine wäßrige Lösung der freien Säure erhalten, die nach Eindampfen und Trocknen im Vakuum bei 40 °C zu einem kristallisierenden Öl führt.

Analog wurden hergestellt :

(3-Sulfopropyl)-(3-methyl-4-chlorphenyl)-ether (Ether (3)),
Fp. 43-50 °C ;
(3-Sulfopropyl)-(4-methylphenyl)-ether (Ether (4)),
Fp. 74-75 °C ;
(3-Sulfopropyl)-(1-naphthyl)-ether (Ether (5)),
Fp. 48-60 °C ;
(3-Sulfopropyl)-(3-chlorphenyl)-ether (Ether (6)) ;
(3-Sulfopropyl)-(3-nitrophenyl)-ether (Ether (7)) ;
(3-Sulfobutyl)-(1-naphthyl)-ether (Ether (8)) ;
Fp. 86-87 °C ;
(4-Sulfobutyl)-(3-methylphenyl)-ether (Ether (9)) ;
(3-Sulfobutyl)-(3-methylphenyl)-ether (Ether (10)).

Das erfindungsgemäße Verfahren und seine Varianten wird durch die folgenden Beispiele näher erläutert :

Beispiel 1

Anhydro-2-methyl-3-(3-sulfopropyl)-benzthiazoliumhydroxid, Fp. 277-279 °C.

2-Methylbenzthiazol wurde mit der gleichen Gewichtsmenge an Ether (1) in der 10-fachen Menge Anisol 7 Stunden gekocht. Nach dem Abkühlen wurde abgesaugt und mit Aceton nachgewaschen. Ausbeute : 60,3 %.

Beispiel 2

Anhydro-2-methyl-3-(3-sulfopropyl)-naphto-[1,2-d]-oxazoliumhydroxid, Fp. 270-273 °C.

a) 2-Methylnaphtho[1,2-d]-oxazol wurde mit Ether (1) im Molverhältnis 1 : 1 3 Stunden auf 180 °C erhitzt. Das in Ethanol aufgenommene abgekühlte Reaktionsgemisch kristallisierte über Nacht aus. Ausbeute : 60 %.

Aus der Mutterlauge kann weiteres Quartärsalz gewonnen weden.

b) Wie a) mit Ether (2) anstelle von Ether (1). Ausbeute : 51 %.
c) Wie a) mit Ether (3) anstelle von Ether (1). Ausbeute : 50 %.
d) Wie a) mit Ether (4) anstelle von Ether (1).

Beispiel 3

Anhydro-2,5,6-trimethyl-3-(3-sulfopropyl)-benzthiazoliumhydroxid, Fp. 291 °C.

Analog Beispiel 2 aus 2,5,6-Trimethylbenzthiazol und Ether (1) in 5 Stunden bei 180 °C. Ausbeute : 61 %.

Beispiel 4

Anhydro-2-methyl-3-(3-sulfopropyl)-naphtho-[1,2-d]-thiazoliumhydroxid, Fp. 273-275 °C.

Analog Beispiel 2 aus 2-Methylnaphtho-[1,2-d]-thiazol und Ether (2) im Molverhältnis 1 : 1,5 in 5 Stunden bei 180 °C. Ausbeute : 16 %.

Beispiel 5

Anhydro-1,2-dimethyl-5,6-dichlor-3-(3-sulfopropyl)-benzimidazoliumhydroxid, Fp. 300 °C.

Analog Beispiel 2 aus 1,2-Dimethyl-5,6-dichlorbenzimidazol und Ether (2) in 3 Stunden bei 180 °C, Ausbeute : 70 %.

Beispiel 6

Anhydro-3-(3-sulfopropyl)-4,5-benzo-3′-ethyl-5′-phenyl-oxacarbocyaninhydroxid.

a) 2-Methylnaphtho-[1,2-d]-oxazol (1/200 Mol) wurde mit Ether (1) im Molverhältnis 1 : 2 3 Stunden auf 180 °C erhitzt. Das Reaktionsgemisch wurde in 25 ml Ethanol aufgenommen und nach Zugabe von 1/200 Mol 2-(2-Phenyliminoethyliden)-5-phenyl-3-ethylbenzoxazol, 0,5 ml Acetanhydrid und 2 ml Triethylamin 5 Minuten bei 50 °C gerührt. Nach Stehen über Nacht wurde der Farbstoff abgesaugt und mit Ethanol gewaschen. Ausbeute : 88 % Absorptionsmaximum in Methanol 506 nm, Fp. 267-268 °C.

b) Wie a) mit Ether 2 ; 68 % Ausbeute.

c) Wie a) mit Ether 3 ; 64 % Ausbeute.

d) Wie a) mit Ether 4 ; 60 % Ausbeute.

e) Wie a) mit Ether 6 ; 52 % Ausbeute.

## Beispiel 7

2-[2-(3-Ethyl-2-thioxo-4-oxo-5-thiazolidinyliden)-ethyliden]-3-(3-sulfopropyl)-5-methyl-6-methoxybenzselenazol, Triethylaminsalz.

a) 1/200 Mol 2,5-Dimethyl-6-methoxybenzselenazol wurden mit 0,075 Mol Ether (2) 3 Stunden auf 180 °C erhitzt. Die Schmelze wurde in 10 ml Ethanol aufgenommen, mit 1,1 g 5-Ethoxymethylen-3-ethylrhodanin und 2 ml Triethylamin versetzt. Die Reaktion war nach 5 Minuten bei 50 °C beendet. Ausbeute : 68 %; Absorptionsmaximum : 544 nm.

b) Wie a) durch 3-stündige Quaternierung bei 160 °C in Gegenwart von 0,5 ml Acetanhydrid ; Ausbeute : 49 %.

## Beispiel 8

Anhydro-3-ethyl-3′-(3-sulfobutyl)-5,5′-diphenyl-oxacarbocyaninhydroxid.

a) 0,005 Mol 5-Phenylbenzoxazol wurden mit 0,01 Mol Ether (10) 3 Stunden auf 180 °C erhitzt. Die erhaltene Masse wurde in 10 ml Ethanol aufgenommen, mit 0,005 Mol 2-(2-Phenyliminoethyliden)-3-ethyl-5-phenyl-benzoxazol und 0,5 ml Acetanhydrid sowie 2 ml Triethylamin versetzt und 5 Minuten bei 50 °C gerührt. Ausbeute : 70 % ; Absorptionsmaximun : 496 nm.

b) Wie a) durch Quarternierung in Gegenwart von 0,5 ml Acetanhydrid 3 Stunden bei 160 °C ; Ausbeute : 69 %.

## Beispiel 9

Anhydro-3,3′-bis-(3-sulfopropyl)-4,5benzothiacyaninhydroxid, Triethylaminsalz.

0,005 Mol 2-Mercaptobenzthiazol wurden mit 0,075 Mol Ether (2) 5 Stunden auf 160 °C erhitzt. Das hierbei gebildete S,N-Bis-(3-sulfopropyl)-Derivat ließ man dann durch Lösen des Reaktionsgemisches in 20 ml Ethanol mit 1,6 g Anhydro-2-methyl-3-(3-sulfopropyl)-naphtho-[1,2-d]-thiazolium-hydroxid in Gegenwart von 2 ml Triethylamin 10 Minuten bei 60 °C reagieren. Ausbeute : 55 %; Absorptionsmaximum in Methanol : 442 nm.

## Beispiel 10

3-Ethyl-5-[3-(3-sulfopropyl)-5-methylthio-1,3,4-thiadiazolin-2-yliden]-rhodanin, Pyridinsalz.

0,1 Mol 2,5-Bis-methylthio-1,3,4-thiadiazol wurden mit 0,2 Mol Ether (2) 3 Stunden auf 140 °C erhitzt. Das Reaktionsprodukt wurde in 50 ml Pyridin aufgenommen und nach Zugabe von 0,1 Mol 3-Ethylrhodanin bis 80 °C noch 5 Stunden bei Raumtemperatur gerührt. Ausbeute : 52 %; Absorptionsmaximum in Wasser : 422 nm.

## Beispiel 11

Anhydro-1-ethyl-1′-(3-sulfopropyl)-2,2′-cyaninhydroxid.

0,005 Mol Chinaldin wurden mit 0,01 Mol Ether (2) 3 Stunden auf 180 °C erhitzt. Nach Abkühlen auf 60 °C gab man 15 ml Ethanol und 0,005 Mol 1-Ethyl-2-ethylthiochinolinium-ethylsulfat und 2 ml Triethylamin hinzu. Die Reaktion war nach 20 Minuten bei 60 °C beendet. Der Farbstoff wurde mit wäßrigem Aceton ausgefällt. Ausbeute : 10 % ; Absorptionsmaximum in Methanol : 525 nm.

## Beispiel 12

Anhydro-2-methyl-5-phenyl-3-(3-sulfopropyl)-benzoxazoliumhydroxid.

a) 0,005 Mol 2-Methyl-5-phenylbenzoxazol wurden mit 0,075 Mol Ether (2) und 0,5 ml Essigsäureanhydrid 3 Stunden auf 180 °C erhitzt. Nach dem Abkühlen wurden 10 ml Essigsäureethylester und 4 ml 1-Propanol zwecks Kristallisation hinzugefügt. Ausbeute : 85 % ; Fp. 280-283 °C.

b) 12,9 g Ether (1) wurden 2 Stunden auf 140 °C erhitzt. Nach Zugabe von 10,5 g 2-Methyl-5-phenylbenzoxazol wurde das Gemisch weiter 3 Stunden auf 175 °C erhitzt. Nach dem Abkühlen wurde mit Ethanol aufgearbeitet. Ausbeute : 13,9 g (84 %) ; Fp. 228-290 °C.

Beispiel 13

Anhydro-2-methyl-5-chlor-3-(4-sulfobutyl)-benzthiazoliumhydroxid.

3,6 g 2-Methyl-5-chlorbenzthiazol wurden mit 7,2 g Ether (9) 4 Stunden auf 175 °C erhitzt. Das Reaktionsgemisch wurde mit Aceton aufgearbeitet. Ausbeute : 74 % ; Zers.: 295 °C.

Beispiel 14

Anhydro-2,5,6-Trimethyl-3-(4-sulfobutyl)-benzthiazoliumhydroxid.

Analog Beispiel 13 aus 2,5,6-Trimethylbenzthiazol und Ether (9). Ausbeute : 74 % ; Zers. : 293-295 °C.

**Ansprüche**

1. Verfahren zur Herstellung von Sulfoalkylquartärsalzen tertiärer Amine, dadurch gekennzeichnet, daß ein tertiäres Amin mit einem Sulfoakyl-arylether der folgenden Formel

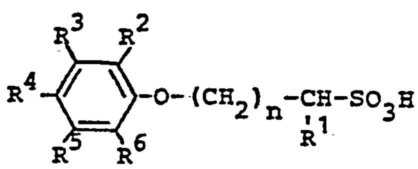

(I)

worin bedeuten

$R^1$ Wasserstoff oder Methyl ;

$R^2$, $R^3$, $R^4$, $R^5$, $R^6$ gleich oder verschieden, und zwar Wasserstoff, Alkyl mit 1 oder 2 C-Atomen, Halogen, Alkoxy, Cyan oder Nitro (wobei jedoch nicht mehr als zwei Nitrogruppen am Phenylring vorhanden sind) ; oder $R^2$ und $R^3$ oder $R^3$ und $R^4$ bedeuten jeweils zusammen die zur Vervollständigung eines ankondensierten 5- oder 6-gliedrigen Ringes erforderlichen Ringglieder ;

n = 1, 2 oder 3 ;

bei einer Temperatur zwischen 100 und 250 °C umgesetzt wird.

2. Verfahren nach Anspruch 1, dadurch gekennzeichnet, daß ein tertiäres Amin der folgenden Formel umgesetzt wird :

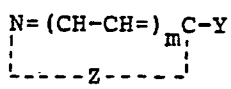

worin bedeuten

Y Wasserstoff, Halogen, eine gesättigte oder ungesättigte, aliphatische Gruppe mit bis zu 6 Kohlenstoffatomen, gegebenenfalls substituiert ; Alkoxy ; Alkylthio oder Mercapto ;

Z die zur Vervollständigung einer heterocyclischen Gruppe mit mindestens einem 5- oder 6-gliedrigen heterocyclischen Ring erforderlichen Ringglieder ;

m 0 oder 1.

3. Verfahren nach den Ansprüchen 1 oder 2, dadurch gekennzeichnet, daß die Umsetzung bei einer Temperatur zwischen 140 und 180 °C durchgeführt wird.

4. Verfahren nach Anspruch 3, dadurch gekennzeichnet, daß die Umsetzung in Gegenwart eines Lösungsmittels durchgeführt wird.

5. Verfahren nach Anspruch 3, dadurch gekennzeichnet, daß die Umsetzung in Gegenwart von Acetanhydrid durchgeführt wird.

6. Verfahren nach Anspruch 3, dadurch gekennzeichnet, daß zunächst der Sulfoalkyl-arylether auf die Reaktionstemperatur erhitzt wird, bevor das tertiäre Amin zugefügt wird.

**Claims**

1. Process for the preparation of sulphoalkyl quaternary salts of tertiary amines, characterised in that a tertiary amine is reacted with a sulphoalkylaryl ether of the following formula

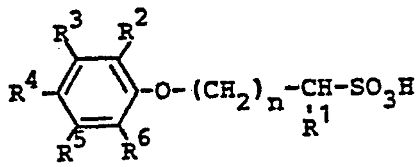

(I)

wherein

$R^1$ denotes hydrogen or methyl ;

$R^2$, $R^3$, $R^4$, $R^5$ and $R^6$ are identical or different and denote hydrogen, alkyl with 1 or 2 C atoms, halogen, alkoxy, cyano or nitro (not more than two nitro groups being however present in the phenyl ring) ; or $R^2$ and $R^3$ or $R^3$ and $R^4$ denote, in each case together, the ring members necessary to complete a condensed 5- or 6-membered ring ; and

n = 1, 2 or 3 ;

at a temperature between 100 and 250 °C.

2. Process according to Claim 1, characterised in that a tertiary amine of the following formula is reacted

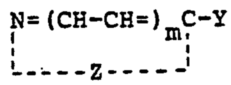

wherein

Y denotes hydrogen, halogen, or a saturated or unsaturated aliphatic group with up to 6 carbon atoms which is optionally substituted ; alkoxy ; or alkylthio or mercapto ;

Z denotes the ring members necessary to complete a heterocyclic group having at least one 5- or 6-membered heterocyclic ring ; and

m denotes 0 or 1.

3. Process according to Claims 1 or 2, characterised in that the reaction is carried at a temperature between 140 and 180 °C.

4. Process according to Claim 3, characterised in that the reaction is carried out in the presence of a solvent.

5. Process according to Claim 3, characterised in that the reaction is carried out in the presence of acetic anhydride.

6. Process according to Claim 3, characterised in that the sulphoalkylaryl ether is first heated to the reaction temperature before the tertiary amine is added.

**Revendications**

1. Procédé pour la fabrication de sels sulfoalkylquaternaires d'amines tertiaires, caractérisé en ce que l'on fait réagir une amine tertiaire à une température comprise entre 100 et 250 °C avec un éther sulfoakyl-arylique de formule suivante

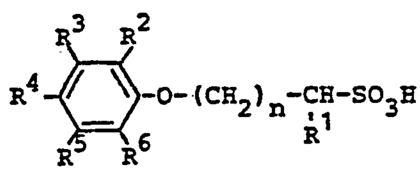

(I)

dans laquelle

$R^1$ représente l'hydrogène ou un groupe méthyle ;

$R^2$, $R^3$, $R^4$, $R^5$, $R^6$ sont identiques ou différents et représentent l'hydrogène, un groupe alkyle ayant 1 ou 2 atomes de carbone, un halogène, un groupe alcoxy, cyano ou nitro (mais, dans ce cas, il n'y a pas plus de deux groupes nitro sur le noyau phényle) ; ou bien $R^2$ et $R^3$ ou $R^3$ et $R^4$ représentent chaque fois ensemble les chaînons nécessaires pour compléter un noyau à 5 ou 6 chaînons accolé ;

n = 1, 2 ou 3.

2. Procédé selon la revendication 1, caractérisé en ce que l'in fait réagir une amine tertiaire de formule suivante

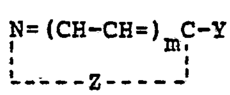

dans laquelle

Y représente l'hydrogène, un halogène, un groupe aliphatique saturé ou insaturé jusqu'en $C_6$, éventuellement substitué ; alcoxy ; alkylthio ou mercapto ;

Z représente les chaînons nécessaires pour compléter un groupe hétérocyclique ayant au moins un noyau hétérocyclique à 5 ou 6 chaînons ;

m est égal à 0 ou 1.

3. Procédé selon la revendication 1 ou 2, caractérisé en ce que la réaction est effectuée à une température entre 140 et 180 °C.

4. Procédé selon la revendication 3, caractérisé en ce que la réaction est effectuée en présence d'un solvant.

5. Procédé selon la revendication 3, caractérisé en ce que la réaction est effectuée en présence d'anhydride acétique.

6. Procédé selon la revendication 3, carcatérisé en ce que l'on chauffe d'abord l'éther sulfoalkylarylique à la température de réaction, avant d'y ajouter l'amine tertiaire.